# FASCICULE DE BREVET EUROPEEN

(11) **EP 0 661 037 B1**
(45) Date de publication et mention de la délivrance du brevet: **24.07.1996**
(21) Numéro de dépôt: 94402919.8
(22) Date de dépôt: 16.12.1994
(51) Int. Cl.: A61K 7/00

(54) **Composition pour lutter contre le vieillissement, agissant simultanément sur les couches superficielles et profondes de la peau, son utilisation**
Mittel gegen Hautalterung, das gleichzeitig auf die Hautoberschichten und Tiefschichten wirkt
Composition against skinaging acting simultaneously on the superficial and deep skinlayers

(30) Priorité: 30.12.1993 FR 9315867
(43) Date de publication de la demande: 05.07.1995
(73) Titulaire: L'OREAL, F-75008 Paris (FR)
(72) Inventeur: Ribier, Alain, F-75001 Paris (FR); Simonnet, Jean-Thierry, F-75011 Paris (FR); Girerd, Florence, F-75020 Paris (FR); Gagnebien-Cabanne, Françoise, F-92320 Chatillon (FR)
(74) Mandataire: Lhoste, Catherine

(56) Documents cités:
- EP-A- 0 559 502
- WO-A-93/15708
- FR-A- 2 408 387
- SOAP, COSMETICS, CHEMICAL SPECIALTIES, vol.69, no.7, 793, US page 77 'formulation ideas'
- INTERNATIONAL JOURNAL OF PHARMACEUTICS, vol.62, no.1, 1990, NL pages 75 - 79 GABRIJELCIC ET AL. 'evaluation of liposomes as drug carriers into the skin by one-dimentional epr imaging'

## Description

La présente invention a pour objet une composition pour le traitement cosmétique ou dermatologique des imperfections ou affections de la peau, y compris le cuir chevelu. Elle se rapporte plus particulièrement à une composition pour lutter contre le vieillissement, comprenant au moins un actif véhiculé par au moins deux catégories distinctes de vésicules lipidiques.

L'invention se rapporte aussi à une utilisation de cette composition pour lutter contre le vieillissement de la peau, et à un procédé cosmétique de traitement contre le vieillissement de la peau.

On connaît de nombreux exemples de compositions cosmétiques ou dermatologiques destinées au traitement de la peau, présentant un ou des actifs adaptés au traitement de la peau, encapsulés dans des vésicules ou sphérules lipidiques (appelées aussi liposomes).

On entend par vésicules ou sphérules lipidiques des particules formées d'une membrane constituée par un ou plusieurs feuillets concentriques, ces feuillets comportant une ou plusieurs couches bimoléculaires de lipides amphiphiles encapsulant une phase aqueuse. La phase aqueuse peut contenir des substances actives hydrosolubles et les couches bimoléculaires de lipides amphiphiles peuvent contenir des substances actives lipophiles.

Ces sphérules ont généralement un diamètre moyen compris entre 10 nm et 5000 nm.

Parmi les nombreux documents publiés concernant cette matière, on peut citer le certificat d'addition français 2408387 qui décrit une composition à base de dispersions aqueuses de sphérules lipidiques ioniques ou non ioniques encapsulant au moins une substance active. Plus précisément, ce document décrit des compositions contenant au moins deux dispersions de sphérules contenant des actifs différents, dans le but d'obtenir un système mixte, c'est-à-dire un système où l'on associe une première dispersion de sphérules contenant une première catégorie de substance active à une seconde dispersion de sphérules contenant une autre catégorie de substance active, qui permet aux deux catégories de substances d'agir simultanément au moment du traitement et d'obtenir éventuellement un effet synergique qui ne se produirait pas si l'on faisait agir successivement et séparément ces deux catégories de substances.

La demanderesse a maintenant mis au point des compositions cosmétiques et/ou dermatologiques pour lutter contre le vieillissement, permettant l'action simultanée de deux actifs différents et permettant, de plus, à ces actifs d'agir dans des zones différentes de la peau, c'est-à-dire dans les couches superficielles et dans les couches profondes de la peau, augmentant de ce fait très nettement l'efficacité de ces compositions et l'effet complémentaire ou synergique des actifs d'antivieillissement mis en oeuvre.

La demanderesse a également mis au point des compositions cosmétiques ou dermatologiques pour lutter contre le vieillissement, permettant au même actif d'agir simultanément dans les couches superficielles et dans les couches profondes de la peau, assurant un traitement plus complet et donc plus efficace du désordre dont elle souffre.

La composition selon l'invention se rapporte aussi bien à la lutte contre l'apparition du vieillissement que celle contre les marques de vieillissement existantes, comme les rides et que la protection de la peau contre les irradiations lumineuses.

On sait qu'au cours du processus de vieillissement, il apparaît différents signes sur la peau, très caractéristiques de ce vieillissement, se traduisant notamment par une modification de la structure et de la fonction cutanée. Ce vieillissement est de nature physiologique mais il est également photoinduit, c'est-à-dire dû à l'exposition répétée de la peau à la lumière et, par conséquent, à la formation de radicaux libres oxygénés par action de cette lumière sur les constituants de la peau.

Les principaux signes cliniques de vieillissement cutané sont notamment les suivants apparition de rides profondes en augmentation avec l'âge. On constate en particulier une désorganisation du "grain" de la peau, c'est-à-dire que le micro-relief est moins régulier et présente un caractère anisotrope.

Par ailleurs, le teint de la peau est généralement modifié, il apparaît plus pâle et plus jaune, ce qui semble être dû essentiellement à une désorganisation de la microcirculation (moins d'hémoglobine au niveau du derme papillaire). De nombreuses taches colorées apparaissent en surface, ce qui est dû à une mélanogénèse altérée. Il existe sur certaines zones des irritations diffuses et parfois des télangiectasies.

Un autre signe clinique de vieillissement est l'aspect sec et rêche de la peau qui est dû essentiellement à une desquamation plus importante, ces squames en diffractant les rayons lumineux participent aussi à l'aspect un peu gris du teint.

On constate enfin une perte de fermeté et de tonicité de la peau qui, comme pour les rides, s'explique du moins en partie par une atrophie dermique et épidermique ainsi qu'un applatissement de la formation dermoépidermique.

On constate donc que les signes cliniques de vieillissement cutané résultent essentiellement d'un dysfonctionnement des principaux mécanismes biologiques intervenant au niveau de la peau.

Aussi, les compositions selon l'invention sont aptes à traiter les rides déjà existantes, à prévenir le vieillissement de la peau et à la protéger en supprimant la formation de radicaux libres.

Il est bien connu que la peau est constituée de couches superficielles, le *Stratum* *Corneum*, et de couches profondes, I'épiderme vivant et le derme. Or, on ne savait pas dans l'art antérieur délivrer spécifiquement tel actif dans les couches superficielles et, simultanément, le même ou tel autre actif dans les couches profondes.

La présente invention a pour objet une composition pour lutter contre le vieillissement, agissant simultanément sur les couches superficielles et profondes de la peau, caractérisée en ce qu'elle comprend une première dispersion de vésicules lipidiques aptes à pénétrer dans les couches profondes de la peau et contenant au moins un actif choisi parmi les agents hydratants, antiradicaux libres, kératolytiques, les protides, les enzymes, les agents antiélastase et anticollagénase, les dérivés d'acide gras, pour traiter ces couches profondes et une deuxième dispersion de vésicules lipidiques aptes à pénétrer dans les couches superficielles de la peau et contenant au moins un actif choisi parmi les agents kératolytiques, tenseurs, hydratants, restructurants de surface et antiradicaux libres, pour traiter ces couches superficielles.

Selon un mode de réalisation particulier, les actifs contenus dans la première dispersion de vésicules et dans la deuxième sont les mêmes.

La demanderesse a utilisé un moyen de tri des vésicules permettant à l'homme de l'art de sélectionner aisément les vésicules lipidiques aptes à véhiculer l'actif dans les couches profondes de la peau, appelées vésicules de profondeur, et celles aptes à véhiculer l'actif dans les couches superficielles de la peau, appelées vésicules de surface.

Ce tri s'effectue sur la base de la constante de diffusion D d'une sonde introduite dans les vésicules. Cette sonde est l'ASL [l'iodure de N-(1-oxyl-2,2,6,6-tétraméthyl-4-pipéridinyl)-N-diméthyl-N-hydroxyéthylammonium] de formule :

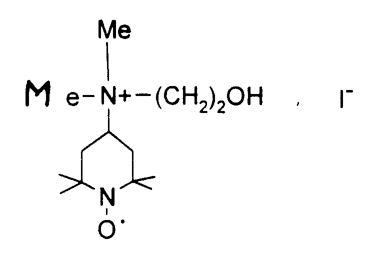

Les vésicules pour lesquelles la constante de diffusion D de la sonde dans le *Stratum Corneum* est > 1.10⁻⁷ cm² s⁻¹ sont les vésicules aptes à pénétrer dans les couches profondes de la peau.

Les vésicules pour lesquelles la constante de diffusion D de la sonde dans le *Stratum Corneum* est < 1.10⁻⁷ cm² s⁻¹ sont les vésicules aptes à véhiculer l'actif dans les couches superficielles de la peau.

Les vésicules de la première catégorie, dites de profondeur, sont en général à l'état fluide à température ambiante (autour de 20 °C), et celles de la seconde catégorie, dites de surface, sont en général à l'état gélifié à la température ambiante. Le moyen de reconnaître l'état des vésicules consiste à déterminer la température de transition de phase (lamellaire fluide-gel) du lipide principal constituant leur membrane, par analyse thermique différentielle (ATD).

D'autres caractéristiques de ces vésicules sont en relation avec leur aptitude à délivrer l'actif plus ou moins en profondeur dans la peau. C'est en particulier le cas du taux d'encapsulation.

Le glucose est un marqueur classiquement utilisé pour ce type de détermination (cf. notamment Liposomes a practical approach de R.R.C. New, IRL Press (1990), p. 125-136).

Le taux d'encapsulation est exprimé par le volume de solution de glucose, encapsulée dans les vésicules, mesuré en µl par rapport à l'unité de poids (mg) des lipides constituant la membrane. Ce taux d'encapsulation est déterminé immédiatement après l'étape de séparation du glucose libre et du glucose encapsulé (T₀) ainsi que vingt-quatre heures après cette séparation (T₂₄ heures).

La différence entre ces 2 déterminations successives illustre la perméabilité des vésicules vis-à-vis du glucose encapsulé, ce qu'on peut encore appeler leur potentiel d'encapsulation.

La première catégorie de vésicules (délivrant l'actif dans les couches profondes de la peau) présente un fort potentiel d'encapsulation des petites molécules hydrosolubles classiquement modélisées par le glucose, ce potentiel d'encapsulation se maintenant au moins 24 heures. La seconde catégorie de vésicules (délivrant l'actif dans les couches superficielles de la peau) ne retient pas le glucose à l'état encapsulé pendant le même temps.

Les lipides principaux constituant les vésicules de la première catégorie (actif délivré en profondeur) comportent au moins une chaîne grasse linéaire et saturée, de longueur allant de 16 à 30 atomes de carbone, tels que les phospholipides hydrogénés (de plantes ou d'oeuf), les phospholipides synthétiques saturés tels que la dipalmitoyl-phosphatidylcholine, les alkyléthers ou alkylesters de polyols à une, deux ou trois chaînes grasses par molécule. Ces lipides sont utilisés seuls ou en mélange.

Les lipides principaux constituant les vésicules de la seconde catégorie (actif délivré en surface) sont choisis en particulier dans le groupe comprenant des lipides ioniques tels que notamment les phospholipides naturels à base de plantes ou d'oeuf, contenant des chaînes grasses insaturées ayant de 16 à 30 atomes de carbone ; des lipides non ioniques tels que les alkyléthers ou alkylesters de polyols comportant une ou plusieurs chaînes grasses par molécule, dont au moins une chaîne grasse de longueur inférieure à 16 atomes de carbone, tels que le lauryl polyglycéryl-6-cétéaryl glycol éther, décrit en détail dans la demande de brevet FR 92-09603 déposée par L'Oréal.

On peut de façon connue incorporer dans la phase lipidique constituant la membrane lipidique des vésicules, au moins un additif choisi dans le groupe formé des stérols (phytostérols, cholestérol, phytostérols polyoxyéthylénés) ; des alcools, diols et triols à longue chaîne (phytanetriol), des amines à longue chaîne et leurs dérivés ammonium quaternaire des esters phosphoriques d'alcools gras et leurs sels alcalins (Na, K) tels que le dicétylphosphate, le dicétylphosphate de sodium, les alkylsulfates (cétylsulfate de sodium), les sels alcalins du cholestérol sulfate ou du cholestérol phosphate, le sel de sodium de l'acide phosphatidique, les lipoaminoacides et leurs sels tels que les acylglutamates de sodium.

On peut citer comme exemple de vésicules de la première catégorie (délivrant l'actif dans les couches profondes de la peau) les vésicules obtenues à partir des lipides suivants (nom CTFA):
- A/ cholestérol / lipoaminoacide caséique notamment dans un rapport pondéral 45/45/10 (où A est un cétyléther de triglycéryle commercialisé par la société Chimex sous le nom Chimexane NL);
- B/ cholestérol / dicétylphosphate notamment dans un rapport pondéral 60/35/5 (où B est un mélange de mono-, di- et tri-cétyléther de triglycéryle commercialisé par la société Chimex sous le nom Chimexane NT);
- Span 40 (de chez Ici ou Sorbitan palmitate)/ cholestérol / acylglutamate de sodium (commercialisé sous le nom de HS11 par la société Ajinomoto), notamment dans un rapport pondéral 47,5/47,5/5 ;
- Stéarate de PEG 8 / cholestérol / acylglutamate de sodium avec notamment un rapport pondéral 47,5/47,5/5 (où le stéarate de PEG 8 est le polyéthylène glycol à 8 motifs d'oxyde d'éthylène commercialisé par la société Unichema sous le nom Stéarate PEG 400) ;
- Stéarate de PEG 8 / cholestérol / phytanetriol / acylglutamate de sodium avec notamment un rapport pondéral 47,5/20/27,5/5;
- Lécithine hydrogénée / phytostérolpolyoxyéthyléné à 5 motifs d'oxyde d'éthylène notamment dans un rapport pondéral 60/40 ;
- Distéarate de méthylglucose polyoxyéthyléné à 20 motifs d'oxyde d'éthylène / cholestérol / acylglutamate de sodium notamment dans un rapport pondéral 45/45/10 (le distéarate étant par exemple celui vendu sous le nom Glucam E 20 distéarate par Amerchol);
- A / cholestérol / dicétylphosphate avec notamment un rapport pondéral 47,5/47,5/5 ;
- Distéarate de diglycéryle (par exemple celui vendu par Nihon sous le nom Emalex DS G2) /cholestérol / acylglutamate de sodium dans un rapport pondéral 45/45/10 ;
- Mono- et di-stéarate de saccharose (par exemple celui vendu par Grillo sous le nom Grilloten PSE 141 G) / cholestérol / acylglutamate de sodium, notamment dans un rapport pondéral 45/45/10;
- Tristéarate de tétraglycéryle (par exemple celui vendu par Nikkol sous le nom Tetraglyn 3S) / cholestérol / acylglutamate de sodium, notamment dans un rapport pondéral de 45/45/10.

On peut citer comme exemples de vésicules de la deuxième catégorie (délivrant l'actif dans les couches superficielles de la peau) les vésicules obtenues à partir des lipides suivants :
- Lécithine de tournesol ;
- Natipide II (lécithine de soja / éthanol / eau dans un rapport pondéral 60/20/20 commercialisé par Nattermann) ;
- C (lécithine de soja / cholestérol / propylène glycol dans un rapport pondéral 40/30/30 commercialisé par Nattermann sous le nom NAT 50 PG) ;
- D / dimyristylphosphate notamment dans un rapport pondéral 95/5 (où D est un éther de lauryl polyglycéryl-6-cétéarylglycol commercialisé par la société Chimex sous le nom Chimexane NS).

Le tableau I ci-après donne pour certaines vésicules obtenues à partir des lipides ci-dessus, la constante de diffusion D de l'ASL dans le *Stratum Corneum* et dans l'épiderme/derme ainsi que le taux d'encapsulation du glucose, et la température de transition de phase du lipide principal constituant la membrane. La constante de diffusion a été mesurée pour une concentration en ASL encapsulé de 0,35 % en poids par rapport au poids total de la composition.

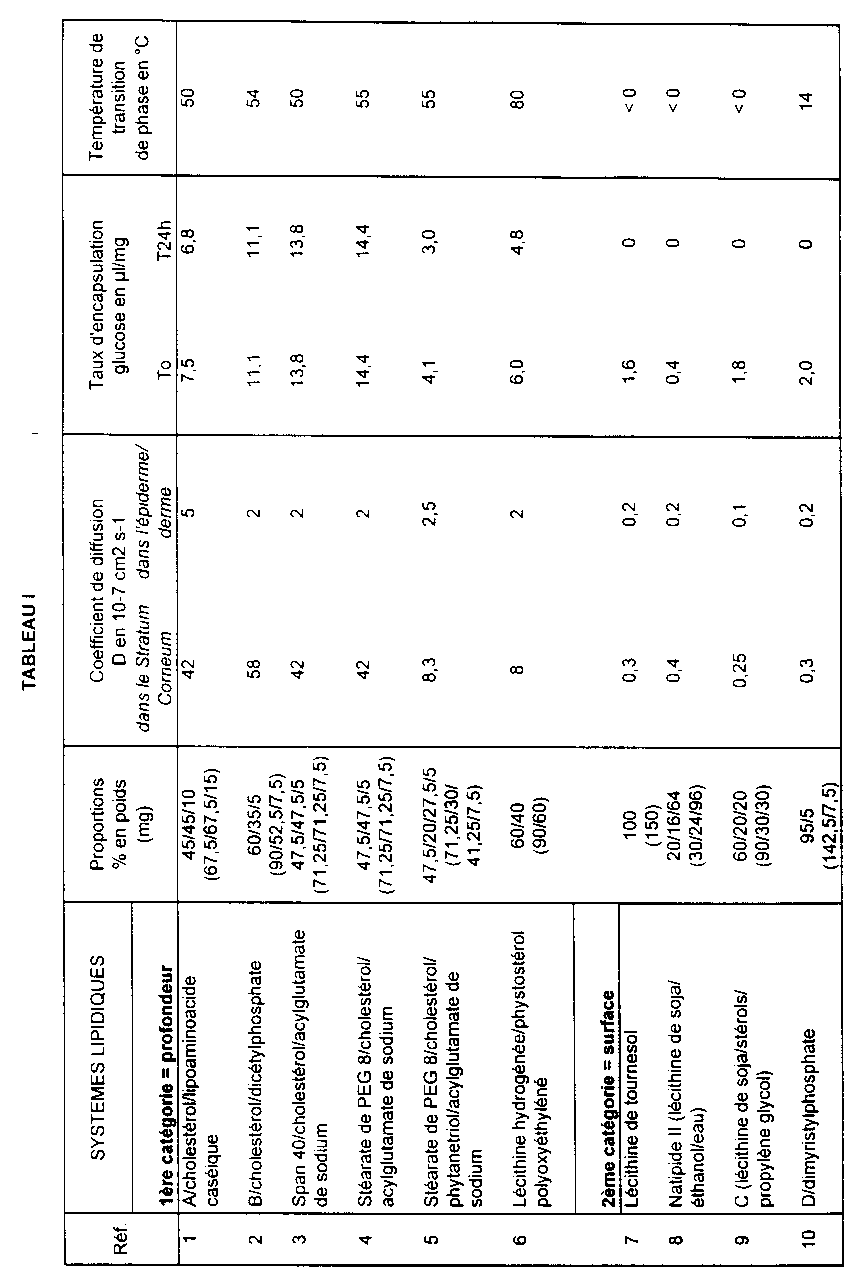

La mesure de la constante de diffusion D s'effectue par combinaison de deux méthodes utilisant une sonde paramagnétique, l'ASL : la résonance paramagnétique électronique (RPE) unidimensionnelle et périodique d'une part et l'imagerie cinétique RPE d'autre part. Ces deux méthodes sont décrites respectivement dans les articles International Journal of Pharmaceutics, 62 (1990) p. 75-79, Elsevier de V. Gabrijelcic et al. "Evaluation of liposomes as drug carriers into the skin by one-dimensional EPR imaging" et Periodicum Biologorum vol. 93, n° 2, p. 245-246, (1991) de V. Gabrijelcic et al. "Liposome entrapped molecules penetration into the skin measured by nitroxide reduction kinetic imaging".

La mesure du taux d'encapsulation s'effectue comme décrit dans le document RRC New cité précédemment et celle de la température de transition de phase comme décrit ci-dessus.

Avantageusement, on utilise simultanément plusieurs actifs dans chaque catégorie de vésicules, présentant la même fonction et/ou conférant à la peau, en surface et en profondeur, le même type d'effet ; les actifs de surface et de profondeur sont donc complémentaires.

Les actifs de profondeur et de surface utilisables dans l'invention sont ceux classiquement utilisés dans le domaine cosmétique ou dermatologique.

Les actifs de profondeur sont choisis parmi les agents hydratants, antiradicaux libres, kératolytiques, les protides, les dérivés d'acide gras, les enzymes, les agents antiélastase et anticollagénase.

Comme agents hydratants, on peut citer le lactate de sodium, les polyols, et en particulier la glycérine , le mannitol et les acides aminés.

Comme agents participant à une action d' antiradicaux libres, on peut citer les dérivés de l'acide phosphonique, l'acide éthylènediamine tétracétique et ses sels tels que le sel de sodium, la guanosine, la superoxydismutase, le tocophérol (vitamine E) et ses dérivés (acétate), I'éthoxyquine, la lactoferrine, la lactoperoxydase, et les dérivés nitroxydes.

Comme agents kératolytiques de profondeur, on peut citer les alpha-hydroxy acides dérivés de fruits tels que l'acide glycolique, l'acide lactique, l'acide citrique, l'acide mandélique et leurs mélanges ; les dérivés d'acide salicylique tels que l'acide n-octanoyl-5-salicylique ou l'acide n-dodécanoyl-5 salicylique, le rétinol (vitamine A) et ses dérivés.

Comme protides, on peut citer les dérivés peptidiques , les protéines (de blé ou de soja) et leurs hydrolysats. Comme dérivés d'acide gras, on peut citer les phospholipides polyinsaturés dont les phospholipides d'acide gras essentiels de poulpe.

Comme agents antiélastase, on peut citer les peptides de graines de légumineuses tels que ceux commercialisés par les laboratoires Sériobiologiques de Nancy sous la référence Parelastyl. Comme agents anticollagénase, on peut citer les inhibiteurs de métalloprotéase tels que l'acide éthylènediamine (EDTA) et la cystéine.

Les enzymes utilisables comme actif de profondeur sont notamment les enzymes de réparation de l'ADN.

Les actifs de surface sont choisis parmi les agents kératolytiques, tenseurs, hydratants, restructurants de surface et antiradicaux libres.

Comme agents kératolytiques de surface, on peut citer les alpha-hydroxy acides tels que l'acide lactique, l'acide salicylique,l'acide n-octanoyl-5-salicylique.

Comme tenseurs, on peut citer les hydrolysats de protéine (par exemple le soja), tels que ceux commercialisés par la société Silab sous le référence Tensine.

Comme restructurant de surface, on peut citer les extraits peptidiques de soja ou de collagène tels que ceux commercialisés par la société Coletica sous la référence Neptigène II.

Comme hydratants de surface, on peut utiliser les mêmes actifs que ceux de profondeur cités précédemment.

Comme antiradicaux libres, on peut citer le tocophérol (vitamine E) et ses dérivés (acétate), les superoxyde dismutases, l'éthoxyquine, la guanosine, le lactopéroxydase, le glutathion peroxydase, les extraits végétaux à activité antiradicalaire tels que l'extrait aqueux de germe de blé commercialisé par la société Silab sous la référence Detoxiline.

Les actifs de surface et de profondeur peuvent représenter de 0,02 % à 10 % du poids total de la composition. De plus, les deux catégories de vésicules peuvent contenir d'autres actifs cosmétiques tels que les oligoéléments, les sucres, etc.

Les compositions selon l'invention peuvent présenter toutes les formes galéniques normalement utilisées pour une application topique telles que les gels aqueux, les émulsions, les lotions, les pommades, les sérums et plus particulièrement les gouttelettes d'huile dispersées par les vésicules telles que décrites dans les brevets français FR-A-2485921 et FR-A-2490504.

De façon connue, dans les compositions de l'invention on peut trouver en plus des vésicules, une huile végétale, minérale, siliconée ou synthétique, dispersée dans une phase aqueuse et également des adjuvants hydrophiles comme les gélifiants, les antioxydants, les conservateurs, les opacifiants, des adjuvants lipophiles tels que les huiles essentielles et les parfums, des pigments et des charges comme décrit dans les brevets français ci-dessus. Par exemple des billes de polyéthylène peuvent être ajoutées pour assurer une action nettoyante (scrub). L'huile dispersée peut représenter de 2 % à 40 % en poids par rapport au poids total de la composition et les adjuvants peuvent représenter, au total, de 0,1 % à 10 % en poids.

L'invention a aussi pour objet une utilisation de la composition définie précédemment pour la préparation d'une pommade destinée à lutter contre le vieillisement ainsi qu'à un procédé pour lutter contre le vieillissement, consistant à appliquer sur la peau cette composition.

D'autres caractéristiques et avantages de l'invention ressortiront mieux de la description qui suit, donnée à titre illustratif et non limitatif.

### A) Obtention de vésicules lipidiques renfermant l'ASL

Les lipides constitutifs de la paroi des vésicules sont pesés et dissous dans 10 ml de méthanol. La solution alcoolique est alors transvasée dans un flacon rond de 50 ml à col rodé, que l'on place ensuite sur un évaporateur rotatif, de telle façon que le contenu soit thermostaté à la température de 30 °C. L'évaporation est poussée jusqu'au dépôt d'un film sec de lipides sur les parois du flacon.

3 ml d'une solution aqueuse 0,01 molaire d'ASL sont alors ajoutés dans le ballon qui est ensuite secoué à la main pendant environ 10 minutes, soit à la température ambiante (20 °C) pour les vésicules du tableau I référencées 7 à 10, soit à la température de 50 °C pour les vésicules référencées 1 à 6 du tableau I. On laisse alors le milieu s'équilibrer à température ambiante, pendant 2 heures, puis on place la dispersion dans un sac de dialyse et au contact de 500 ml d'eau distillée. La dialyse s'effectue pendant une nuit. Après une nuit, l'eau est changée et la dialyse est poursuivie pendant 4 heures supplémentaires.

Un fil de coton de 0,3 mm d'épaisseur est alors mis à tremper dans la dispersion de vésicules puis mis au contact d'une coupe de peau provenant d'une oreille de porc fraîchement récupérée dans un abattoir destiné à l'approvisionnement alimentaire.

L'échantillon d'oreille prélevé est rincé à l'eau et découpé en tranches de 1 mm d'épaisseur, 5 mm de large et 10 mm de long puis placé dans une cellule de maintien. Les mesures de diffusion de l'ASL dans la peau sont effectuées dans les 24 heures suivant le prélèvement de peau.

### B) Obtention de la composition cosmétique

### 1° Obtention des vésicules de première catégorie (diffusant en profondeur)

On prépare les vésicules (de profondeur) selon une méthode usuelle de co-fusion des différents constituants (voir tableau I) de la membrane choisis. Ainsi, on fond le constituant membranaire ayant le point de fusion T_{f} le moins élevé. On ajoute les autres constituants membranaires y compris les actifs puis on homogénéise sous agitation moyenne et enfin on hydrate partiellement en maintenant la température de fusion T_{f} définie ci-dessus.

A la pâte obtenue, on ajoute une solution aqueuse d'au moins un premier actif, pour le traitement en profondeur. Une turbine est actionnée pendant 1 h 30 pour bien hydrater, en maintenant la température T_{f}. On rajoute au milieu réactionnel un ou plusieurs autres actifs pour le traitement en profondeur, on homogénéise et diminue la température du milieu jusqu'à la température ambiante (20 °C).

### 2° Obtention de vésicules de seconde catégorie (diffusant en surface)

On introduit à température ambiante (20 °C) et par simple agitation une solution aqueuse d'un (ou plusieurs) second actif pour le traitement en surface dans le mélange choisi de constituants devant former la membrane des vésicules de surface (voir tableau I). On obtient ainsi des vésicules de surface encapsulant le second actif de surface.

### 3° Obtention de la composition "double liposomes"

Au milieu contenant les vésicules de profondeur, on ajoute la phase grasse (les huiles) de la composition et on la disperse (à température ambiante) sous agitation. On mélange alors le milieu réactionnel obtenu à celui contenant les vésicules de surface. Eventuellement, on ajoute alors les adjuvants tels que des conservateurs, un gélifiant que l'on peut neutraliser si nécessaire par une base (triéthanolamine ou soude) et des parfums, etc... .

Le produit obtenu se présente sous forme d'une crème blanche, douce et onctueuse, utilisable dans le domaine cosmétique et/ou dermatologique selon la nature des actifs (de surface et de profondeur) choisis.

On donne ci-après des exemples particuliers de compositions cosmétiques conformes à l'invention.

### EXEMPLE 1 : Crème double liposomes, anti-rides

| - *Préparation A : liposomes de profondeur :* | |
|---|---|
| Stéarate de PEG 8/cholestérol/acylglutamate de sodium dans un rapport pondéral 47,5/47,5/5 | 11,26 g |
| Acétate de tocophérol | 1,00 g |
| Glycérine | 5,63 g |
| Hydrolysat de protéine de soja (actif) | 0,37 g |
| Sel disodique de l'acide éthylènediamine-tétracétique (séquestrant) | 0,19 g |
| Eau déminéralisée | qsp 100 g |

| - *Préparation B : liposomes de surface :* | |
|---|---|
| Natipide II commercialisé par la société Natterman | 33,33 g |
| Glycérine (actif) | 33,33 g |
| Collagène natif marin (actif) | 8,33 g |
| Eau déminéralisée | qsp 100 g |

| - *Composition double liposomes :* | |
|---|---|
| Préparation A | 26,65 g |
| Préparation B | 9,00 g |
| Huile végétale | 16,40 g |
| Huile de silicone volatile | 4,00 g |
| Palmitate de rétinyle | 0,6 g |
| Parfum | 0,4 g |
| Acide citrique | 0,02 g |
| Microsphères de copolymère de chlorure de vinylidène | 0,20 g |
| Conservateurs | 1,18 g |
| Polymère carboxyvinylique (gélifiant) | 0,50 g |
| Soude | 0,15 g |
| Eau déminéralisée | qsp 100 g |

### EXEMPLE 2 : Crème double liposomes, antiradicaux libres

| - *Préparation A : Liposomes de profondeur :* | |
|---|---|
| Stéarate de PEG 8/cholestérol/acylglutamate de sodium dans un rapport pondéral 47,5/47,5/5 | 10,33 g |
| alpha-tocophérol | 1,00 g |
| Glycérine | 13,77 g |
| Guanosine | 0,30 g |
| Sel disodique de l'acide éthylènediamine-tétracétique (actif) | 0,17 g |
| Eau déminéralisée | qsp 100 g |

| - *Préparation B : liposomes de surface :* | |
|---|---|
| Natipide II commercialisé par la société Natterman | 35,29 g |
| Extrait aqueux de germes de blé (actif) commercialisé par la société Silab sous la référence Detoxiline | 5,88 g |
| Glycérine | 35,30 g |
| Eau déminéralisée | qsp 100 g |

| - *Composition double liposomes :* | |
|---|---|
| Préparation A | 29,05 g |
| Préparation B | 8,50 g |
| Huile végétale | 20,50 g |
| Huile de silicone volatile | 3,50 g |
| Parfum | 0,40 g |
| Acide citrique | 0,02 g |
| Conservateurs | 1,18 g |
| Polymère carboxyvinylique (gélifiant) | 0,50 g |
| Soude | 0,15 g |
| Eau déminéralisée | qsp 100 g |

### EXEMPLE 3 : Crème double liposomes, anti-âge

| - *Préparation A : Liposomes de profondeur :* | |
|---|---|
| Stéarate de PEG 8/cholestérol/acylglutamate de sodium dans un rapport pondéral 47,5/47,5/5 | 11,52 g |
| Glycérine | 5,76 g |
| Sel disodique de l'acide éthylènediamine-tétracétique (séquestrant) | 0,19 g |
| Mélange d'alpha-hydroxy acides de fruits (acides lactique/glycolique/citrique : 35/15/8) (actif) | 1,92 g |
| Eau déminéralisée | qsp 100 g |

| - *Préparation B : Liposomes de surface :* | |
|---|---|
| Natipide II commercialisé par la société Natterman | 35,29 g |
| Acide lactique (actif) | 5,88 g |
| Eau déminéralisée | qsp 100 g |

| - *Composition double liposomes :* | |
|---|---|
| Préparation A | 26,05 g |
| Préparation B | 8,50 g |
| Huile végétale | 17,00 g |
| Huile de silicone volatile | 4,00 g |
| Parfum | 0,40 g |
| Conservateurs | 1,18 g |
| Polymère carboxyvinylique (gélifiant) | 0,50 g |
| Soude | 0,15 g |
| Eau déminéralisée | qsp 100 g |

### EXEMPLE 4 : Crème double liposomes, anti-âge

| - *Préparation A : Liposomes de profondeur :* | |
|---|---|
| Stéarate de PEG 8/cholestérol/acylglutamate de sodium dans un rapport pondéral 47,5/47,5/5 | 11,26 g |
| Acétate de tocophérol | 0,37 g |
| Acide n-octanoyl-5-salicylique (actif) | 0,37 g |
| Glycérine | 5,63 g |
| Sel disodique de l'acide éthylènediamine-tétracétique | 0,19 g |
| Eau déminéralisée | qsp 100 g |

| - *Préparation B : Liposomes de surface :* | |
|---|---|
| Natipide II commercialisé par la Société Natterman | 36,58 g |
| Acide salicylique (actif) | 2,44 g |
| Glycérine | 36,6 g |
| Eau déminéralisée | qsp 100 g |

| - *Composition double liposomes :* | |
|---|---|
| Préparation A | 26,65 g |
| Préparation B | 8,20 g |
| Huile végétale | 17,00 g |
| Huile de silicone volatile | 4,00 g |
| Parfum | 0,40 g |
| Acide citrique | 0,02 g |
| Conservateurs | 1,70 g |
| Polymère carboxyvinylique (gélifiant) | 0,50 g |
| Soude | 0,15 g |
| Eau déminéralisée | qsp 100 g |

## Revendications

1. Composition pour lutter contre le vieillissement, agissant simultanément sur les couches superficielles et profondes de la peau, caractérisée en ce qu'elle comprend une première dispersion de vésicules lipidiques aptes à pénétrer dans les couches profondes de la peau et contenant au moins un actif choisi parmi les agents hydratants, antiradicaux libres, kératolytiques, les protides, les agents antiélastase et anticollagénase, les enzymes, les dérivés d'acide gras, pour traiter ces couches profondes et une deuxième dispersion de vésicules lipidiques aptes à pénétrer dans les couches superficielles de la peau et contenant au moins un actif choisi parmi les agents kératolytiques, tenseurs, hydratants, restructurants de surface, antiradicaux libres, pour traiter ces couches superficielles.

2. Composition selon la revendication 1, caractérisée en ce que les vésicules de la première dispersion assurent une diffusion d'ASL [(iodure de N-(1-oxyl-2,2,6,6-tétraméthyl-4-pipéridinyl)-N-diméthyl-N hydroxyéthylammonium], dans le *Stratum Corneum*, > 1.10⁻⁷ cm²/s et en ce que les vésicules de la seconde dispersion assurent une diffusion d'ASL dans le *Stratum Corneum* < 1.10⁻⁷ cm²/s.

3. Composition selon la revendication 1 ou 2, caractérisée en ce que les vésicules de la première dispersion sont à l'état fluide à température ambiante et les vésicules de la seconde dispersion sont à l'état gélifié à température ambiante.

4. Composition selon l'une quelconque des revendications 1 à 3, caractérisée en ce que les vésicules de la première dispersion assurent une encapsulation du glucose pendant au moins 24 heures et en ce que les vésicules de la seconde dispersion assurent une encapsulation du glucose pendant moins de 24 heures.

5. Composition selon l'une quelconque des revendications 1 à 4, caractérisée en ce que les vésicules de la première dispersion sont formées de lipides comportant au moins une chaîne grasse linéaire et saturée ayant de 16 à 30 atomes de carbone.

6. Composition selon l'une quelconque des revendications 1 à 5, caractérisée en ce que les vésicules de la première dispersion sont formées d'au moins un lipide choisi parmi les phospholipides naturels hydrogénés, les phospholipides synthétiques saturés, les alkyléthers de polyols à au moins une chaîne linéaire grasse, les alkylesters de polyols à au moins une chaîne grasse, et leurs mélanges.

7. Composition selon l'une quelconque des revendications 1 à 6, caractérisée en ce que les vésicules de la première dispersion sont formées d'au moins un lipide choisi parmi:
Cétyléther de triglycéryle / cholestérol / lipoaminoacide caséique ;
Mélange de mono-, di- et tri-cétyléther de triglycéryle / cholestérol / dicétylphosphate ;
Cétyléther de triglycéryle / cholestérol / dicétylphosphate ;
Sorbitan palmitate / cholestérol / acylglutamate de sodium ;
Stéarate de PEG 8 / cholestérol / acylglutamate de sodium ;
Distéarate de diglycéryle / cholestérol / acylglutamate de sodium ;
Mono- et di-stéarate de saccharose / cholestérol / acylglutamate de sodium ;
Stéarate de PEG 8 / cholestérol / phytanetriol / acylglutamate de sodium ;
Distéarate de méthylglucose polyoxyéthyléné à 20 moles d'oxyde d'éthylène / cholestérol / acylglutamate de sodium ;
Lécithine hydrogénée / phytostérol polyoxyéthyléné ;
Tristéarate de tétraglycéryle / cholestérol / acylglutamate de sodium.

8. Composition selon l'une quelconque des revendications 1 à 7, caractérisée en ce que les vésicules de la seconde dispersion sont formées de lipides choisis parmi les phospholipides ioniques naturels contenant des chaînes grasses insaturées ayant de 16 à 30 atomes de carbone, les alkyléthers ou alkylesters de polyols comportant une ou plusieurs chaînes grasses par molécule, dont au moins une chaîne grasse de longueur inférieure à 16 atomes de carbone et leurs mélanges.

9. Composition selon l'une quelconque des revendications 1 à 8, caractérisée en ce que les vésicules de la seconde dispersion sont formées d'au moins un lipide choisi parmi :
Lécithine de tournesol ;
Lécithine de soja / éthanol / eau ;
Lécithine de soja / cholestérol / propylène glycol ;
Ether de lauryl polyglycéryl-6-cétéarylglycol / dimyristylphosphate.

10. Composition selon l'une quelconque des revendications 1 à 9, caractérisée en ce que l'actif de la première dispersion et celui de la seconde dispersion assurent la même fonction et/ou le même type d'effet.

11. Composition selon l'une quelconque des revendications 1 à 10, caractérisée en ce que les actifs de première et seconde dispersion sont les mêmes.

12. Composition selon l'une quelconque des revendications 1 à 11, caractérisée en ce que l'actif contenu dans la première dispersion est choisi parmi la glycérine, les dérivés d'acide phosphonique, l'acide éthylènediaminetétracétique et ses sels, les alpha-hydroxyacides et leurs sels, les dérivés d'acide salicylique, les dérivés peptidiques, les hydrolysats de protéine, les phospholipides polyinsaturés, l'acide n-octanoyl-5-salicylique, le tocophérol et ses dérivés, le rétinol et ses dérivés, les superoxydismutases, la guanosine, la lactoperoxydase et la lactoferrine.

13. Composition selon l'une quelconque des revendications 1 à 12, caractérisée en ce que l'actif contenu dans la seconde dispersion est choisi parmi l'acide salicylique et ses dérivés, l'acide n-octanoyl-5-salicylique, les alpha-hydroxyacides, les hydrolysats de protéines, les dérivés du collagène, la glycérine, le tocophérol et ses dérivés, les superoxyde dismutases, I'éthoxyquine, la guanosine, la lactopéroxydase.

14. Composition selon l'une quelconque des revendications 1 à 13, caractérisée en ce qu'elle contient, en outre, une phase huileuse dispersée dans une phase aqueuse.

15. Composition selon l'une quelconque des revendications 1 à 14, caractérisée en ce qu'elle contient, en outre, des adjuvants hydrophiles ou lipophiles.

16. Utilisation de la composition selon l'une quelconque des revendications 1 à 15, pour la préparation d'une pommade destinée au traitement du vieillissement.

17. Procédé cosmétique de traitement du vieillissement, caractérisé en ce qu'il consiste à appliquer sur la peau une composition selon l'une quelconque des revendications 1 à 15.

## Claims

1. Composition for combating ageing, which acts simultaneously on the surface layers and deep layers of the skin, characterized in that it comprises a first dispersion of lipid vesicles which are capable of penetrating into the deep layers of the skin and which contain at least one active agent chosen from moisturizing agents, anti-free-radical agents, keratolytic agents, protides, anti-elastase agents and anti-collagenase agents, enzymes, and fatty acid derivatives, for treating these deep layers, and a second dispersion of lipid vesicles which are capable of penetrating into the surface layers of the skin and which contain at least one active agent chosen from keratolytic agents, tensioning agents, moisturizing agents, surface-restructuring agents and anti-free-radical agents, for treating these surface layers.

2. Composition according to Claim 1, characterized in that the vesicles of the first dispersion provide a diffusion of ASL [N-(1-oxido-2,2,6,6-tetramethyl-4-piperidyl)-N,N-dimethyl-N-hydroxyethylammoniumiodide] in the *stratum corneum* of > 1.10⁻⁷ cm²/s and in that the vesicles of the second dispersion provide a diffusion of ASL in the *stratum corneum* of < 1.10⁻⁷ cm²/s.

3. Composition according to Claim 1 or 2, characterized in that the vesicles of the first dispersion are in the fluid state at room temperature and the vesicles of the second dispersion are in the gelled state at room temperature.

4. Composition according to any one of Claims 1 to 3, characterized in that the vesicles of the first dispersion provide an encapsulation of glucose for at least 24 hours, and in that the vesicles of the second dispersion provide an encapsulation of glucose for less than 24 hours.

5. Composition according to any one of Claims 1 to 4, characterized in that the vesicles of the first dispersion are formed of lipids composed of at least one linear and saturated fatty chain having from 16 to 30 carbon atoms.

6. Composition according to any one of Claims 1 to 5, characterized in that the vesicles of the first dispersion are formed of at least one lipid chosen from natural hydrogenated phospholipids, saturated synthetic phospholipids, polyol alkyl ethers having at least one linear fatty chain, polyol alkyl esters having at least one fatty chain, and mixtures thereof.

7. Composition according to any one of Claims 1 to 6, characterized in that the vesicles of the first dispersion are formed of at least one lipid chosen from:
Triglyceryl cetyl ether/cholesterol/casein lipoamino acid;
Mixture of triglyceryl mono-, di- and tricetyl ether/cholesterol/dicetyl phosphate;
Triglyceryl cetyl ether/cholesterol/dicetyl phosphate;
Sorbitan palmitate/cholesterol/sodium acylglutamate;
PEG 8 stearate/cholesterol/sodium acylglutamate;
Diglyceryl distearate/cholesterol/sodium acylglutamate;
Sucrose mono- and distearate/cholesterol/sodium acylglutamate;
PEG 8 stearate/cholesterol/phytanetriol/sodium acylglutamate;
Polyoxyethylenated methylglucose distearate containing 20 mol of ethylene oxide/cholesterol/sodium acylglutamate;
Hydrogenated lecithin/polyoxyethylenated phytosterol;
Tetraglyceryl tristearate/cholesterol/sodium acylglutamate.

8. Composition according to any one of Claims 1 to 7, characterized in that the vesicles of the second dispersion are formed of lipids chosen from natural ionic phospholipids containing unsaturated fatty chains having from 16 to 30 carbon atoms, polyol alkyl ethers or polyol alkyl esters composed of one or more fatty chains per molecule, including at least one fatty chain with a length of less than 16 carbon atoms, and mixtures thereof.

9. Composition according to any one of Claims 1 to 8, characterized in that the vesicles of the second dispersion are formed of at least one lipid chosen from:
Sunflower lecithin;
Soya lecithin/ethanol/water;
Soya lecithin/cholesterol/propylene glycol;
Lauryl polyglyceryl-6-cetearyl glycol ether/dimyristyl phosphate.

10. Composition according to any one of Claims 1 to 9, characterized in that the active agent of the first dispersion and that of the second dispersion provide the same function and/or the same type of effect.

11. Composition according to any one of Claims 1 to 10, characterized in that the active agents of the first and second dispersions are the same.

12. Composition according to any one of Claims 1 to 11, characterized in that the active agent contained in the first dispersion is chosen from glycerol, phosphonic acid derivatives, ethylenediaminetetraacetic acid and the salts thereof, alpha-hydroxy acids and the salts thereof, salicylic acid derivatives, peptide derivatives, protein hydrolysates, polyunsaturated phospholipids, 5-n-octanoylsalicylic acid, tocopherol and the derivatives thereof, retinol and the derivatives thereof, superoxide dismutases, guanosine, lactoperoxidase and lactoferrin.

13. Composition according to any one of Claims 1 to 12, characterized in that the active agent contained in the second dispersion is chosen from salicylic acid and the derivatives thereof, 5-n-octanoylsalicylic acid, alpha-hydroxy acids, protein hydrolysates, collagen derivatives, glycerol, tocopherol and the derivatives thereof, superoxide dismutases, ethoxyquin, guanosine and lactoperoxidase.

14. Composition according to any one of Claims 1 to 13, characterized in that it additionally contains an oily phase dispersed in an aqueous phase.

15. Composition according to any one of Claims 1 to 14, characterized in that it additionally contains hydrophilic or lipophilic adjuvants.

16. Use of the composition according to any one of Claims 1 to 15 for the preparation of an ointment intended for the treatment of ageing.

17. Cosmetic process for treating ageing, characterized in that it consists in applying to the skin a composition according to any one of Claims 1 to 15.

## Patentansprüche

1. Zusammensetzung gegen Hautalterung, die gleichzeitig in den oberflächlichen und den unteren Hautschichten wirkt,
dadurch **gekennzeichnet,** daß
sie zur Behandlung der unteren Hautschichten eine erste Dispersion von Lipidvesikeln, die in die unteren Hautschichten eindringen können und mindestens einen unter den Hydratisierungsmitteln, Mitteln gegen freie Radikale, Keratolytika, Protidverbindungen wie Proteinen, Peptidverbindungen und deren Hydrolysaten, Mitteln gegen Elastase und Kollagenase, Enzymen und Fettsäurederivaten ausgewählten Wirkstoff und zur Behandlung der oberen Hautschichten eine zweite Dispersion von Lipidvesikeln, die in die oberflächlichen Hautschichten eindringen können und mindestens einen unter Keratolytika, Tensiden, Hydratisierungsmitteln, Mitteln zur Restrukturierung der Oberfläche und Mitteln gegen freie Radikale ausgewählten Wirkstoff enthält.

2. Zusammensetzung nach Anspruch 1,
dadurch gekennzeichnet, daß
die Vesikel der ersten Dispersion eine Diffusionskonstante der Diffusion von ASL (N-(1-Oxyl-2,2,6,6-tetramethylpiperidin-4-yl)-N-dimethyl-N-hydroxyethylammoniumiodid) in das Stratum Corneum von > 1·10⁻⁷ cm²/s und die Vesikel der zweiten Dispersion eine ASL-Diffusion in das Stratum Corneum von < 1·10⁻⁷ cm²/s gewährleisten.

3. Zusammensetzung nach Anspruch 1 oder 2,
dadurch gekennzeichnet, daß
die Vesikel der ersten Dispersion bei Raumtemperatur in einem flüssigen Zustand und die Vesikel der zweiten Dispersion bei Raumtemperatur im Gelzustand vorliegen.

4. Zusammensetzung nach einem der Ansprüche 1 bis 3,
dadurch gekennzeichnet, daß
die Vesikel der ersten Dispersion einen Einschluß von Glucose während mindestens 24 Stunden und die Vesikel der zweiten Dispersion einen Einschluß von Glucose während weniger als 24 Stunden gewährleisten.

5. Zusammensetzung nach einem der Ansprüche 1 bis 4,
dadurch gekennzeichnet, daß
die Vesikel der ersten Dispersion aus Lipiden gebildet sind, die mindestens eine geradkettige und gesättigte Fettsäurekette mit 16 bis 30 Kohlenstoffatomen aufweisen.

6. Zusammensetzung nach einem der Ansprüche 1 bis 5,
dadurch gekennzeichnet, daß
die Vesikel der ersten Dispersion aus mindestens einem Lipid gebildet sind, das unter hydrierten natürlichen Phospholipiden, gesättigten synthetischen Phospholipiden, Alkylethern von Polyolen mit mindestens einer geradkettigen Fettsäurekette und Alkylestern von Polyolen mit mindestens einer Fettsäurekette sowie ihren Gemischen ausgewählt ist.

7. Zusammensetzung nach einem der Ansprüche 1 bis 6,
dadurch gekennzeichnet, daß
die Vesikel der ersten Dispersion aus mindestens einem Lipid gebildet sind, das ausgewählt ist unter:
Triglycerylcetylether/Cholesterin/Casein-Lipoaminosäuren;
einem Gemisch aus Triglycerylmono-, -di- und -tricetylether/Cholesterin/Dicetylphosphat;
Triglycerylcetylether/Cholesterin/Dicetylphosphat;
Sorbitanpalmitat/Cholesterin/Natriumacylglutamat;
PEG-8-Stearat/Cholesterin/Natriumacylglutamat;
Diglyceryldistearat/Cholesterin/Natriumacylglutamat;
Saccharosemono- und -distearat/Cholesterin/Natriumacylglutamat;
PEG-8-Stearat/Cholesterin/Phytantriol/Natriumacylglutamat;
Methylglucosedistearat, das mit 20 mol Ethylenoxid polyethoxyliert ist/Cholesterin/Natriumacylglutamat;
hydriertes Lecithin/polyethoxyliertes Phytosterin;
Tetraglyceryltristearat/Cholesterin/Natriumacylglutamat.

8. Zusammensetzung nach einem der Ansprüche 1 bis 7,
dadurch gekennzeichnet, daß
die Vesikel der zweiten Dispersion aus Lipiden gebildet sind, die unter natürlichen ionischen Phospholipiden, die ungesättigte Fettsäureketten mit 16 bis 30 Kohlenstoffatomen enthalten, Alkylethern oder Alkylestern von Polyolen mit einer oder mehreren Fettsäureketten pro Molekül, wobei mindestens eine Fettsäurekette weniger als 16 Kohlenstoffatome aufweist, und deren Gemischen ausgewählt ist.

9. Zusammensetzung nach einem der Ansprüche 1 bis 8,
dadurch gekennzeichnet, daß
die Vesikel der zweiten Dispersion aus mindestens einem Lipid gebildet sind, das ausgewählt ist unter:
Lecithin aus Sonnenblumen,
Lecithin aus Soja/Ethanol/Wasser,
Lecithin aus Soja/Cholesterin/Propylenglykol,
Polyglyceryl-6-cetearylglykollaurylether/Dimyristylphosphat.

10. Zusammensetzung nach einem der Ansprüche 1 bis 9,
dadurch gekennzeichnet, daß
der Wirkstoff der ersten Dispersion und der zweiten Dispersion die gleiche Wirksamkeit und/oder die gleiche Wirkungsart aufweisen.

11. Zusammensetzung nach einem der Ansprüche 1 bis 10,
dadurch gekennzeichnet, daß
die Wirkstoffe der ersten und zweiten Dispersion identisch sind.

12. Zusammensetzung nach einem der Ansprüche 1 bis 11,
dadurch gekennzeichnet, daß
der in der ersten Dispersion enthaltene Wirkstoff unter Glycerin, Phosphonsäurederivaten, Ethylendiamintetraessigsäure und ihren Salzen, α-Hydroxysäuren und ihren Salzen, Salicylsäurederivaten, Peptidderivaten, Proteinhydrolysaten, mehrfach ungesättigten Phospholipiden, 5-(n-Octanoyl)-salicylsäure, Tocopherol und seinen Derivaten, Retinol und seinen Derivaten, Superoxid-Dismutasen, Guanosin, Lactoperoxidase und Lactoferrin ausgewählt ist.

13. Zusammensetzung nach einem der Ansprüche 1 bis 12,
dadurch gekennzeichnet, daß
der in der zweiten Dispersion enthaltene Wirkstoff unter Salicylsäure und ihren Derivaten, 5-(n-Octanoyl)-salicylsäure, α-Hydroxysäuren, Proteinhydrolysaten, Kollagenderivaten, Glycerin, Tocopherol und seinen Derivaten, Superoxid-Dismutasen, Ethoxyquin, Guanosin und Lactoperoxidase ausgewählt ist.

14. Zusammensetzung nach einem der Ansprüche 1 bis 13,
dadurch gekennzeichnet, daß
sie ferner eine in einer wäßrigen Phase dispergierte Ölphase enthält.

15. Zusammensetzung nach einem der Ansprüche 1 bis 14,
dadurch gekennzeichnet, daß
sie ferner hydrophile oder lipophile Hilfsstoffe enthält.

16. Verwendung der Zusammensetzung nach einem der Ansprüche 1 bis 15 zur Herstellung einer für die Behandlung der Hautalterung bestimmten Salbe.

17. Kosmetisches Verfahren zur Behandlung gegen Alterung,
dadurch gekennzeichnet, daß
es im Auftragen einer Zusammensetzung nach einem der Ansprüche 1 bis 15 auf die Haut besteht.
